# EUROPEAN PATENT APPLICATION

(11) **EP 4 306 632 A1**
(43) Date of publication of application: **17.01.2024**
(21) Application number: 22766595.7
(22) Date of filing: 14.01.2022
(51) Int. Cl.: C12M 3/00

(54) **FLUID CIRCUIT DEVICE AND CELL CULTURE DEVICE**

(30) Priority: 09.03.2021 JP 2021037414
(71) Applicant: TOKYO OHKA KOGYO CO., LTD., Kawasaki-shi, Kanagawa 211 0012 (JP)
(72) Inventor: KUMAZAWA Hirotsugu, Kawasaki-shi, Kanagawa 211-0012 (JP); YAZAWA Tomoyuki, Kawasaki-shi, Kanagawa 211-0012 (JP)
(74) Representative: Plasseraud IP
(86) International application number: PCT/JP2022/001245
(87) International publication number: WO 2022/190627

(57) **Abstract**

This fluid circuit device includes a block that has a guide portion in which a pipe allowing a fluid sent from a pump to circulate therein is attachable and detachable. The guide portion includes at least one of a recessed portion that guides the pipe along a surface of the block, and a hole portion that guides the pipe through the inside of the block.

## Description

### TECHNICAL FIELD

The present invention relates to a fluid circuit device and a cell culture device.

Priority is claimed on Japanese Patent Application No. 2021-037414, filed March 9, 2021, the content of which is incorporated herein by reference.

### BACKGROUND ART

A device for culturing cells by causing a cell culture fluid (for example, a culture solution) to flow inside a flow channel is known.

For example, Patent Document 1 discloses a cell culture device that is provided with a cell culture member including a micro-flow channel, and a circulation device for circulating a culture solution inside the micro-flow channel of the cell culture member. The circulation device includes a reservoir, a circulation pump, a pressure equalization mechanism, and an air-trap/pressurization mechanism. Tubes are connected to respective portions of constituent elements of the circulation device. A circulation path is constituted by connecting the tubes to the respective portions.

For example, Patent Document 2 discloses a cell culture module that includes a holding member in which a cell culture carrier provided with a plurality of wells on a surface thereof is placed, and a lid member which covers a top surface of the holding member. A first recessed portion accommodating the cell culture carrier is formed on the top surface of the holding member. A culture solution supply flow channel having one open end on a bottom surface of the first recessed portion is formed in the holding member. A second recessed portion having a size corresponding to that of the cell culture carrier is formed on a lower surface of the lid member. In the lid member, a flow channel for cell injection, culture solution discharge, and cell collection having one open end on a bottom surface of the second recessed portion, and a flow channel for culture solution discharge having one open end on a side surface of the second recessed portion are formed.

### RELATED ART DOCUMENT

### PATENT DOCUMENT

[Patent Document 1]
   Japanese Unexamined Patent Application, First Publication No. 2015-186474
[Patent Document 2]
   Japanese Unexamined Patent Application, First Publication No. 2015-123028

### SUMMARY

### PROBLEMS TO BE SOLVED BY THE INVENTION

In the case of Patent Document 1, since respective portions of constituent elements of a circulation device are connected through tubes, there is a possibility that problems may occur, such as the tubes being mixed up in a mess, erroneous connection of the tubes, culture solution leakage, and the unnecessary use of a culture solution due to excessively long tubes.

In the case of Patent Document 2, since respective flow channels are formed in a holding member and a lid member, when the holding member and the lid member are reused in terms of cost, there is a possibility of contamination due to a supplied culture solution or secretions or the like from cultured cells adhering to the inside of a flow channel.

For this reason, there is room for improvement in reducing costs by reusing each member and curbing contamination.

In consideration of the foregoing circumstances, an object of the present invention is to provide a fluid circuit device and a cell culture device capable of reducing costs and curbing contamination.

### MEANS FOR SOLVING THE PROBLEM

A fluid circuit device according to an aspect of the present invention includes a block that has a guide portion in which a pipe allowing a fluid sent from a pump to circulate therein is attachable and detachable. The guide portion includes at least one of a recessed portion that guides the pipe along a surface of the block, and a hole portion that guides the pipe through the inside of the block.

According to the present aspect, since the pipe through which a fluid circulates and the blocks are segregated, even when the pipe is contaminated by a fluid, contamination of the block can be avoided and the block can be reused in terms of cost. Furthermore, when the pipe is contaminated, the contamination can be curbed by removing the pipe from the guide portion of the block and attaching an uncontaminated pipe to the guide portion. Therefore, costs can be reduced and contamination can be curbed.

Furthermore, when the pipe is attached to the guide portion of the block, since the pipe can be guided by at least one of the recessed portion of the block and the inside of the block, a situation in which the pipe is mixed up in a mess can be curbed. Therefore, occurrence of erroneous connection of the pipe or leakage of a fluid can be curbed and unnecessary use of a fluid due to an excessively long pipe can be curbed.

In the foregoing fluid circuit device, the block may be formed of a resin.

According to this constitution, the weight can be reduced compared to when the block is formed of a metal.

In the foregoing fluid circuit device, the pipe may be formed of a material with lower drug adsorption compared to the block.

According to this constitution, since a situation in which a drug remains inside the pipe can be curbed, an optimal device for evaluation of a drug can be provided.

In the foregoing fluid circuit device, the block may include a plurality of sub-blocks that are segregated in a manner of being attachable to and detachable from each other.

According to this constitution, the block can be made compact in units of a plurality of sub-blocks constituting it. Furthermore, for instance, even when the block is partially contaminated, since only the contaminated one of the plurality of sub-blocks constituting the block can be discarded and the remainder can be reused, it is suitable for reducing costs and curbing contamination.

In the foregoing fluid circuit device, the pipe may include a plurality of tubes that are provided correspondingly to the plurality of sub-blocks.

According to this constitution, the pipe can be made compact in units of a plurality of tubes constituting it. Furthermore, even when the pipe is partially contaminated, since only the contaminated one of the plurality of tubes constituting the pipe can be discarded and the remainder can be reused, it is suitable for reducing costs and curbing contamination.

In the foregoing fluid circuit device, the plurality of tubes may include a pump connection tube that is connectable to the pump, and a tank connection tube that is connectable to a tank storing the fluid. The plurality of sub-blocks may include a pump block that has a pump-side guide portion in which the pump connection tube is attachable and detachable, and a tank block that has a tank-side guide portion which is connected in a manner of being attachable and detachable with respect to the pump block and in which the tank connection tube is attachable and detachable.

According to this constitution, even when one of the pump connection tube or the tank connection tube is contaminated, only the contaminated one can be discarded and the remainder can be reused. Furthermore, for instance, even when one of the pump block or the tank block is contaminated, only the contaminated one can be discarded and the remainder can be reused.

In the foregoing fluid circuit device, the pump block may have a pump mounting portion in which the pump is mountable. The tank block may have a tank mounting portion in which the tank is mountable.

According to this constitution, the tank is mounted in the tank mounting portion while the pump is mounted in the pump mounting portion when the device is in use, and the pump and the tank can be detached when the device is not in use. Therefore, it is suitable for managing the pump and the tank.

In the foregoing fluid circuit device, the plurality of tubes may further include a coupling tube that allows the pump connection tube and the tank connection tube to be coupled. The plurality of sub-blocks may further include a coupling block that has a coupling guide portion in which the coupling tube is attachable and detachable. The coupling block may be connected in a manner of being attachable and detachable with respect to the pump block and the tank block.

According to this constitution, even when any of the pump connection tube, the coupling tube, and the tank connection tube is contaminated, only the contaminated one can be discarded and the remainder can be reused. Furthermore, for instance, even when any of the pump block, the coupling block, and the tank block is contaminated, only the contaminated one can be discarded and the remainder can be reused. Furthermore, since the pump connection tube and the tank connection tube can be directly connected or can be connected via the coupling tube, the degree of freedom in layout of the pipe is improved. Furthermore, since the pump block and the tank block can be directly connected or can be connected via the coupling block, the degree of freedom in layout of the block is improved.

In the foregoing fluid circuit device, at least one of the plurality of sub-blocks may include a plurality of cassettes that are connected in a manner of being attachable to and detachable from each other. The pipe may include a plurality of tubes that are provided correspondingly to at least one of the plurality of cassettes.

According to this constitution, the sub-blocks can be made compact in units of a plurality of cassettes constituting them. Furthermore, for instance, even when the sub-blocks are partially contaminated, only the contaminated one of the plurality of cassettes constituting the sub-blocks can be discarded and the remainder can be reused. Furthermore, when a plurality of tubes are provided correspondingly to the plurality of cassettes, the pipe can be made compact in units of a plurality of tubes constituting it. Furthermore, even when the pipe is partially contaminated, only the contaminated one of the plurality of tubes constituting the pipe can be discarded and the remainder can be reused.

The foregoing fluid circuit device may further include a joint member that is provided at a part where the plurality of sub-blocks are connected to each other and is connected in a manner of being attachable and detachable with respect to the tubes.

According to this constitution, since the tubes can be easily attached and detached using the joint member, erroneous connection can be reduced and connection work can be made efficient.

A cell culture device according to another aspect of the present invention includes a block that has a guide portion in which a pipe allowing a cell culture fluid to circulate therein is attachable and detachable. The guide portion includes at least one of a recessed portion that guides the pipe along a surface of the block, and a hole portion that guides the pipe through the inside of the block.

According to the present aspect, since the pipe through which a cell culture fluid circulates and the block are segregated, even when the pipe is contaminated by a cell culture fluid, contamination of the block can be avoided and the block can be reused in terms of cost. Furthermore, when the pipe is contaminated, the contamination can be curbed by removing the pipe from the guide portion of the block and attaching an uncontaminated pipe to the guide portion. Therefore, costs can be reduced and contamination can be curbed. Furthermore, cytotoxicity caused by a cell culture fluid remaining inside the pipe can be reduced. Furthermore, when the pipe is attached to the guide portion of the block, since the pipe can be guided by at least one of the recessed portion of the block and the inside of the block, a situation in which the pipe is mixed up in a mess can be curbed. Therefore, occurrence of erroneous connection of the pipe or leakage of a fluid can be curbed and unnecessary use of a fluid due to an excessively long pipe can be curbed.

In the foregoing cell culture device, the block may include a plurality of coupling blocks that are coupled in a manner of being attachable to and detachable from each other. The plurality of coupling blocks may have pipe connection systems that are different from each other in accordance with the purpose of cell culture.

According to this constitution, since the pipe connection systems can be varied in accordance with the purpose of cell culture, an optimal device for cell culture can be provided.

In the foregoing cell culture device, the block may include a supply cassette that has a supply guide portion in which a supply tube capable of supplying the cell culture fluid is attachable and detachable with respect to a culture chip capable of storing the fluid. The supply cassette may be connected in a manner of being attachable and detachable with respect to an upper portion of the culture chip.

According to this constitution, since the supply cassette is connectable to the upper portion of the culture chip when the device is in use and the supply cassette can be detached when the device is not in use, it is suitable for managing the supply cassette. Furthermore, compared to a constitution in which the supply cassette is connected to a lower portion of the culture chip, connection work can be made efficient.

### ADVANTAGE OF THE INVENTION

According to the present invention, it is possible to provide a fluid circuit device and a cell culture device capable of reducing costs and curbing contamination.

### BRIEF DESCRIPTION OF THE DRAWINGS

FIG. 1 is a perspective view of a block constituting a cell culture device of an embodiment.
FIG. 2 is an exploded perspective view of the block of the embodiment divided into a plurality of sub-blocks.
FIG. 3 is a top view of the cell culture device of the embodiment.
FIG. 4 is a view including a cross section IV-IV in FIG. 3.
FIG. 5 is an enlarged view of a pump block in the cell culture device illustrated in FIG. 4.
FIG. 6 is an enlarged view of a tank block in the cell culture device illustrated in FIG. 4.
FIG. 7 is an enlarged view of a coupling block in the cell culture device illustrated in FIG. 4.
FIG. 8 is a top view of the cell culture device according to a first modification example of the embodiment.
FIG. 9 is a top view of the cell culture device according to a second modification example of the embodiment.
FIG. 10 is a view including a cross section corresponding to that in FIG. 4 of the cell culture device according to a third modification example of the embodiment.

### DESCRIPTION OF THE EMBODIMENTS

Hereinafter, an embodiment of the present invention will be described with reference to the drawings. In the following embodiment, as an example of a fluid circuit device, a cell culture device for culturing cells by circulating a cell culture liquid (an example of a fluid) will be described.

### <Cell culture device>

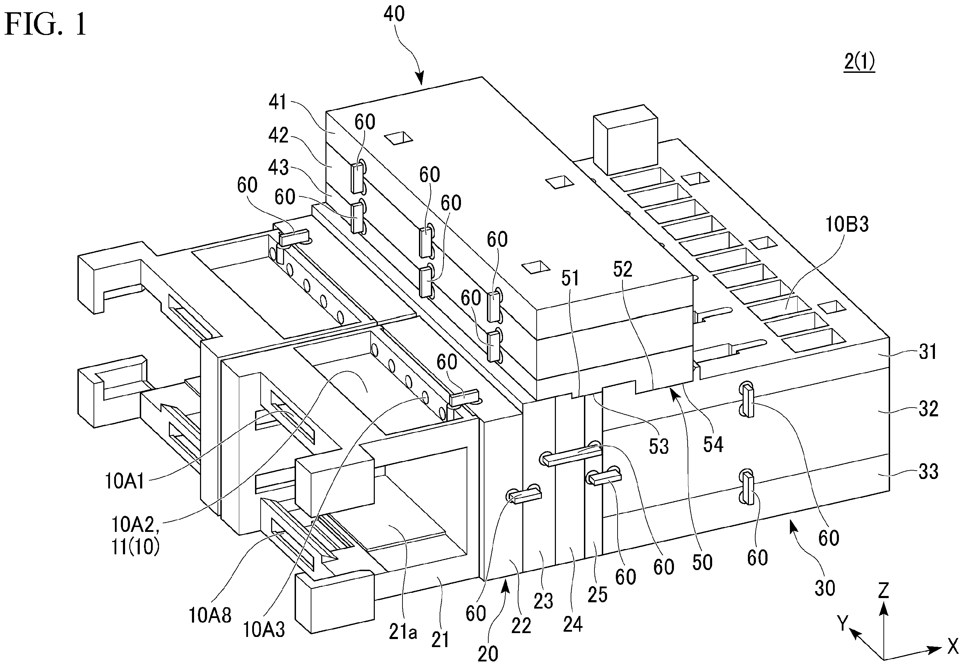

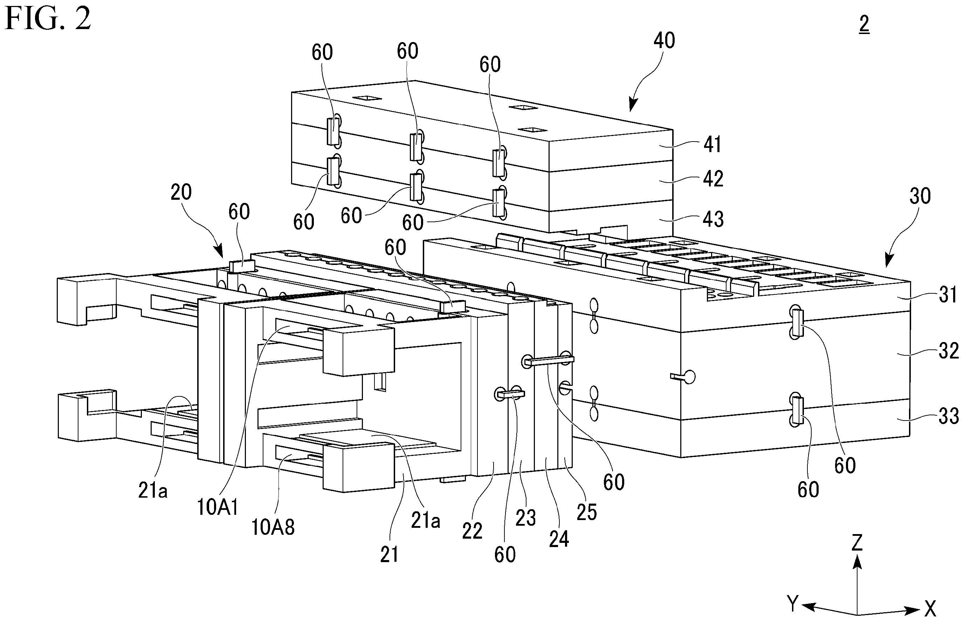

FIG. 1 is a perspective view of a block 2 constituting a cell culture device 1 of the embodiment. FIG. 2 is an exploded perspective view the block 2 of the embodiment divided into a plurality of sub-blocks 20, 30, and 40.

As illustrated in FIG. 1, the cell culture device 1 includes the block 2 that is a block-shaped member. For example, the block 2 is formed of a resin such as acrylic nitrile, butadiene, a styrene copolymer synthetic resin (ABS resin), or polycarbonate (PC).

In the following description, an XYZ orthogonal coordinate system is set, and positional relationships of the members will be described with reference to this XYZ orthogonal coordinate system. A predetermined direction within a horizontal plane will be referred to as an X direction, a direction orthogonal to the X direction within the horizontal plane will be referred to as a Y direction, and a direction orthogonal to each of the X direction and the Y direction (that is, a vertical direction) will be referred to as a Z direction. In the following description, in the X direction, the Y direction, and the Z direction, a side directed to by the arrow in the diagrams will be described as a positive (+) side, a side opposite to that directed to by the arrow will be described as a negative (-) side. A positive Z side corresponds to the upward side in the vertical direction, and a negative Z side corresponds to a downward side in the vertical direction.

In the following description, for example, expressions indicating relative or absolute disposition, such as "parallel", "orthogonal", "center", and "coaxial" not only strictly denote such disposition but also include a tolerance and a relatively displaced state at an angle or a distance to the extent that the same function can be achieved. In the drawings used for the following description, the size of each member is suitably changed such that each member has a recognizable size.

### <Block>

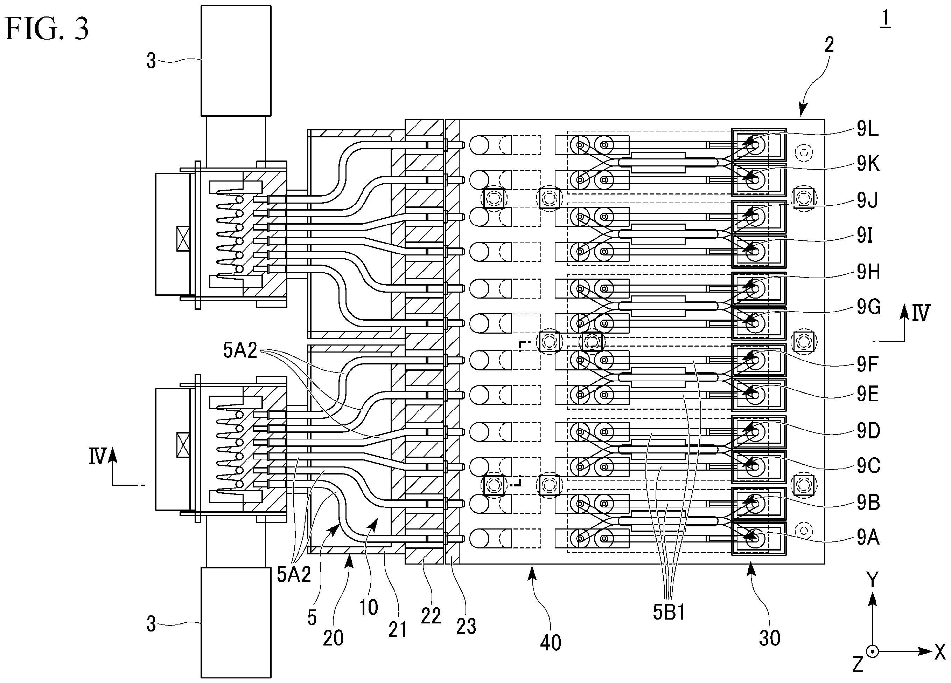

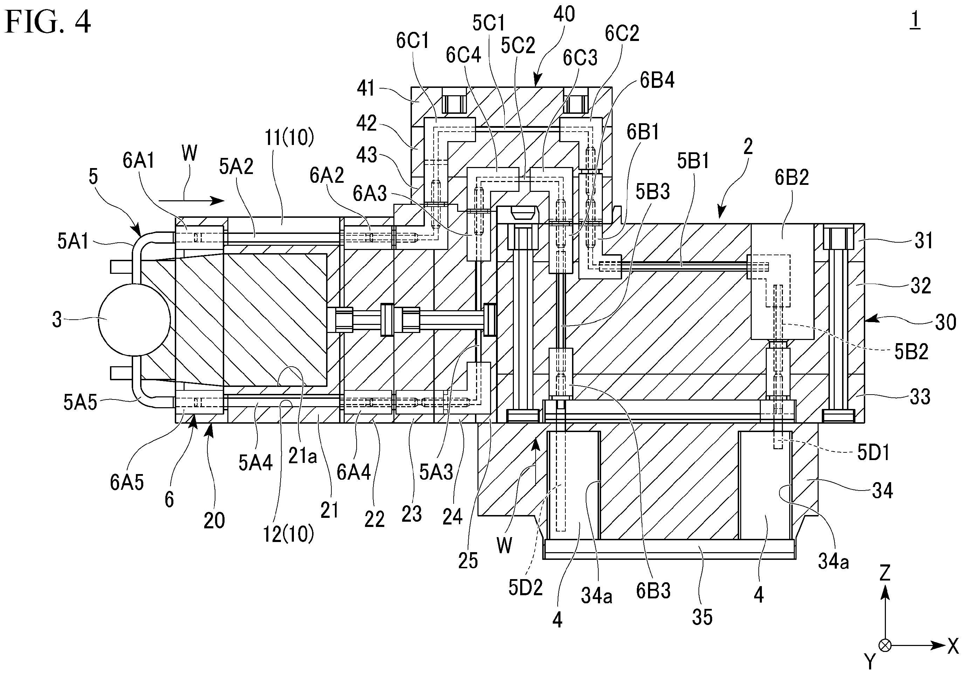

FIG. 3 is a top view of the cell culture device 1 of the embodiment. FIG. 4 is a view including a cross section IV-IV in FIG. 3.

As illustrated in FIG. 3, the block 2 has guide portions 10 in which a pipe 5 allowing a cell culture liquid (which will hereinafter be referred to as "a culture solution") sent from a pump 3 to circulate therein is attachable and detachable. For example, a culture solution includes various kinds of drugs for culturing cells.

As illustrated in FIG. 4, the guide portions 10 include a recessed portion 11 guiding the pipe 5 along a surface of the block 2, and a hole portion 12 guiding the pipe 5 through the inside of the block 2. Here, the recessed portion 11 denotes a part, such as a depression or a groove that can be seen from the external appearance of the block 2, for guiding the pipe 5 along or the like, an outer surface (surface) of the block 2. The hole portion 12 denotes a part, such as a penetration hole or a space that cannot be seen from the external appearance of the block 2, for guiding the pipe 5 through an internal space of the block 2.

As illustrated in FIG. 2, the block 2 includes a plurality of (for example, three in the present embodiment) sub-blocks 20, 30, and 40 segregated in a manner of being attachable to and detachable from each other. The three sub-blocks 20, 30, and 40 are constituted of a pump block 20, a tank block 30, and a coupling block 40.

As illustrated in FIG. 1, the pump block 20 and the tank block 30 are connected in a manner of being attachable to and detachable from each other using fixing members 60 such as lock pins. The coupling block 40 is connected in a manner of being attachable and detachable with respect to the pump block 20 and the tank block 30 by an uneven structure 50 including recessed portions and projecting portions. A connection method using fixing members is not limited to that described above and diverse methods such as screwing and fixing using bands can be employed.

### <Pump block>

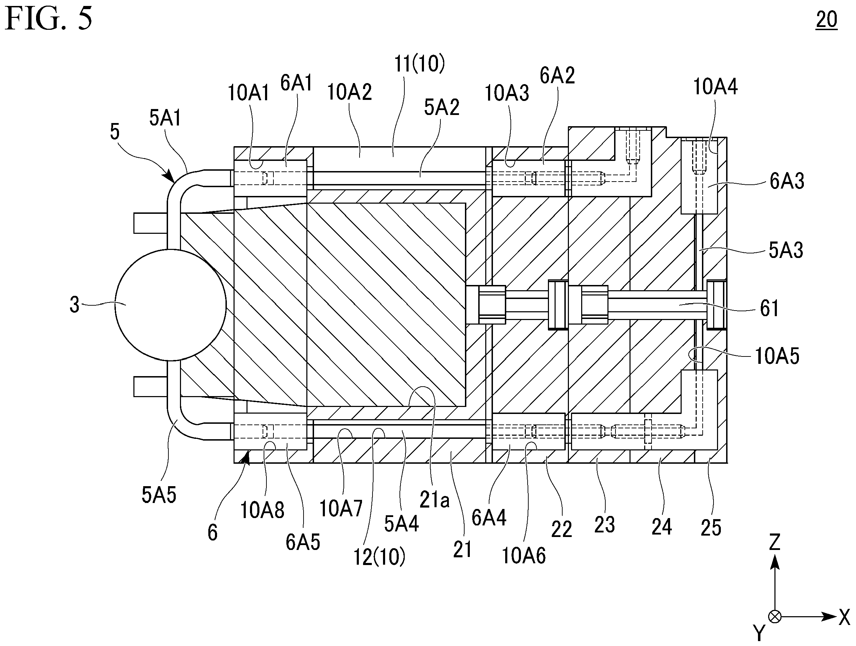

FIG. 5 is an enlarged view of the pump block 20 in the cell culture device 1 illustrated in FIG. 4.

As illustrated in FIG. 5, the pump block 20 has pump-side guide portions 10A1 to 10A8 (an example of guide portions) in which pump connection tubes SA1 to 5A5 constituting the pipe 5 is attachable and detachable. The pump block 20 has pump mounting portions 21a in which the pump 3 can be mounted.

As illustrated in FIG. 2, two pump mounting portion 21a are provided with a gap therebetween in the Y direction. Accordingly, as illustrated in FIG. 3, two pumps 3 can be mounted in the pump block 20. The number of pumps 3 to be installed is not limited to that described above and can be changed in accordance with the required specifications.

As illustrated in FIG. 5, the pump block 20 includes a plurality of (for example, five in the present embodiment) cassettes 21 to 25 connected in a manner of being attachable to and detachable from each other. The five cassettes 21 to 25 constituting the pump block 20 are a first pump cassette 21, a second pump cassette 22, a third pump cassette 23, a fourth pump cassette 24, and a fifth pump cassette 25. The five cassettes 21 to 25 are disposed in the order of the first pump cassette 21, the second pump cassette 22, the third pump cassette 23, the fourth pump cassette 24, and the fifth pump cassette 25 from a negative X side toward a positive X side. The number of cassettes, which constitute the pump block 20, to be installed is not limited to that described above and can be changed in accordance with the required specifications.

The first pump cassette 21 is formed to have a U-shape opening on the negative X side when viewed in the Y direction. The first pump cassette 21 has the pump mounting portion 21a. The pump mounting portion 21a is provided inside the U-shaped opening formed by the first pump cassette 21.

As illustrated in FIG. 2, each of the second pump cassette 22, the third pump cassette 23, the fourth pump cassette 24, and the fifth pump cassette 25 is formed to have a rectangular parallelepiped shape having a long side in the Y direction and having a short side in the Z direction. The lengths of the second pump cassette 22, the third pump cassette 23, the fourth pump cassette 24, and the fifth pump cassette 25 in the Y direction are the same.

The first pump cassette 21 and the second pump cassette 22 are connected in a manner of being attachable to and detachable from each other using the fixing members 60 (in FIG. 2, two fixing members 60 on the positive Z side are illustrated) such as lock pins. The second pump cassette 22 and the third pump cassette 23 are connected in a manner of being attachable to and detachable from each other using the fixing members 60 (in FIG. 2, the fixing member 60 on a negative Y side is illustrated) such as lock pins. The third pump cassette 23 and the fifth pump cassette 25 are connected in a manner of being attachable to and detachable from each other using the fixing members 60 (in FIG. 2, the fixing member 60 on the negative Y side is illustrated) such as lock pins in a state of sandwiching the fourth pump cassette 24 therebetween. As illustrated in FIG. 5, the first pump cassette 21 to the fifth pump cassette 25 are connected in a manner of being attachable to and detachable from each other using fixing members 61 such as bolts. A connection method using fixing members is not limited to that described above and diverse methods such as screwing and fixing using bands can be employed.

### <Tank block>

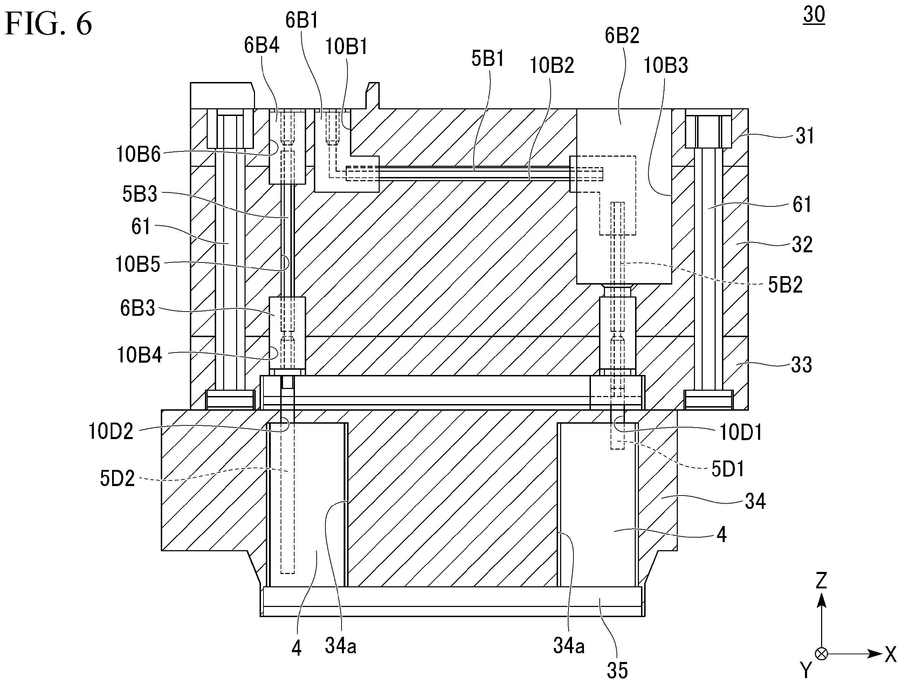

FIG. 6 is an enlarged view of the tank block 30 in the cell culture device 1 illustrated in FIG. 4.

As illustrated in FIG. 6, the tank block 30 has tank-side guide portions 10B1 to 10B6 (an example of guide portions) in which relay tubes 5B1 to 5B3 constituting the pipe 5 is attachable and detachable. As illustrated in FIG. 2, the tank block 30 is formed to have a rectangular parallelepiped shape having a long side in the Y direction and having a short side in the X direction.

As illustrated in FIG. 6, the tank block 30 includes a plurality of (for example, three in the present embodiment) cassettes 31 to 33 connected in a manner of being attachable to and detachable from each other. The three cassettes 31 to 33 constituting the tank block 30 are a first tank cassette 31, a second tank cassette 32, and a third tank cassette 33. The three cassettes 31 to 33 are disposed in the order of the first tank cassette 31, the second tank cassette 32, and the third tank cassette 33 from the positive Z side toward the negative Z side. The number of cassettes, which constitute tank block 30, to be installed is not limited to that described above and can be changed in accordance with the required specifications.

As illustrated in FIG. 2, each of the first tank cassette 31, the second tank cassette 32, and the third tank cassette 33 is formed to have a rectangular parallelepiped shape having a long side in the Y direction and having a short side in the X direction. The lengths of the first tank cassette 31, the second tank cassette 32, and the third tank cassette 33 in the Y direction are the same with respect to the fifth pump cassette 25. The second tank cassette 32 has a larger thickness (length in the Z direction) than each of the first tank cassette 31 and the second tank cassette 32.

The first tank cassette 31 and the second tank cassette 32 are connected in a manner of being attachable to and detachable from each other using the fixing members 60 (in FIG. 2, the fixing member 60 on the negative Y side is illustrated) such as lock pins. The second tank cassette 32 and the third tank cassette 33 are connected in a manner of being attachable to and detachable from each other using the fixing members 60 (in FIG. 2, the fixing member 60 on the negative Y side is illustrated) such as lock pins. As illustrated in FIG. 1, the second tank cassette 32 and the fifth pump cassette 25 are connected in a manner of being attachable to and detachable from each other using the fixing members 60 (in FIG. 1, the fixing member 60 on the negative Y side is illustrated) such as lock pins. As illustrated in FIG. 6, the first tank cassette 31 to the third tank cassette 33 are connected in a manner of being attachable to and detachable from each other using the fixing members 61 such as bolts. A connection method using fixing members is not limited to that described above and diverse methods such as screwing and fixing using bands can be employed.

### <Coupling block>

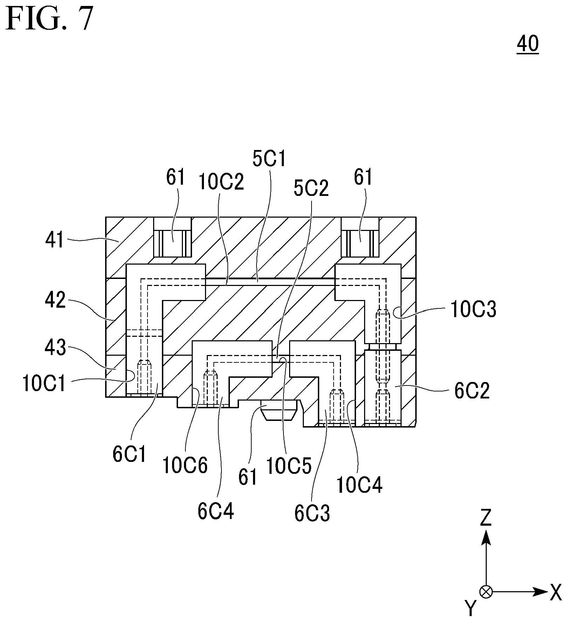

FIG. 7 is an enlarged view of the coupling block 40 in the cell culture device 1 illustrated in FIG. 4.

As illustrated in FIG. 7, the coupling block 40 has coupling guide portions 10C1 to 10C6 in which coupling tubes 5C1 to 5C2 is attachable and detachable. As illustrated in FIG. 2, the coupling block 40 is formed to have a rectangular parallelepiped shape having a long side in the Y direction and having a short side in the X direction. The lengths of the coupling block 40 and the tank block 30 in the Y direction are the same as each other.

As illustrated in FIG. 7, the coupling block 40 includes a plurality of (for example, three in the present embodiment) cassettes 41 to 43 connected in a manner of being attachable to and detachable from each other. The three cassettes 41 to 43 constituting the coupling block 40 are a first couple cassette 41, a second couple cassette 42, and a third couple cassette 43. The three cassettes are disposed in the order of the first couple cassette 41, the second couple cassette 42, and the third couple cassette 43 from the positive Z side toward the negative Z side. The number of cassettes, which constitute the coupling block 40, to be installed is not limited to that described above and can be changed in accordance with the required specifications.

As illustrated in FIG. 2, each of the first couple cassette 41, the second couple cassette 42, and the third couple cassette 43 is formed to have a rectangular parallelepiped shape having a long side in the Y direction and having a short side in the X direction. The lengths of the first couple cassette 41, the second couple cassette 42, and the third couple cassette 43 in the Y direction are the same with respect to the third pump cassette 23, the fourth pump cassette 24, and the fifth pump cassette 25.

The first couple cassette 41 and the second couple cassette 42 are connected in a manner of being attachable to and detachable from each other using the fixing members 60 (in FIG. 2, three fixing members 60 disposed at a part on the negative X side with a gap therebetween in the Y direction are illustrated) such as lock pins. The second couple cassette 42 and the third couple cassette 43 are connected in a manner of being attachable to and detachable from each other using the fixing members 60 (in FIG. 2, three fixing members 60 disposed at a part on the negative X side with a gap therebetween in the Y direction are illustrated) such as lock pins. As illustrated in FIG. 7, the first couple cassette 41 to the third couple cassette 43 are connected in a manner of being attachable to and detachable from each other using the fixing members 61 such as bolts. A connection method using fixing members is not limited to that described above and diverse methods such as screwing and fixing using bands can be employed.

As illustrated in FIG. 1, the third couple cassette 43 is connected in a manner of being attachable and detachable with respect to the pump block 20 and the tank block 30 by the uneven structure 50 including recessed portions and projecting portions. The uneven structure 50 includes a first recessed portion 51 provided at a part on the positive X side and the positive Z side in the pump block 20, a second recessed portion 52 provided at a part on the negative X side and the positive Z side in the tank block 30, a first projecting portion 53 provided at a position corresponding to the first recessed portion 51 in the third couple cassette 43, and a second projecting portion 54 provided at a position corresponding to the second recessed portion 52 in the third couple cassette 43. The shape of the recessed portions and the projecting portions constituting the uneven structure 50 is not limited to that described above and can be changed in accordance with the required specifications.

### <Pipe>

As illustrated in FIG. 4, the pipe 5 is disposed inside and outside the block 2. The pipe 5 is formed of a material with lower drug adsorption compared to the block 2. For example, when the block 2 is formed of an ABS resin, it is preferable that the pipe 5 be formed of a polyether ether ketone resin (PEEK resin) or a fluororesin (for example, polytetrafluoroethylene or the like).

The pipe 5 includes a plurality of tubes SA1 to 5A5, 5B1 to 5B3, 5C1 to 5C2, and 5D1 to 5D2 provided correspondingly to the plurality of sub-blocks 20, 30, and 40. The plurality of tubes 5A1 to 5A5, 5B1 to 5B3, 5C1 to 5C2, and 5D1 to 5D2 include the pump connection tubes 5A1 to 5A5 which are connectable to the pump 3, the relay tubes 5B1 to 5B3 for relaying the pipe, the coupling tubes 5C1 to 5C2 which can couple the pump connection tubes 5A1 to 5A5 and the relay tubes 5B1 to 5B3, and the tank connection tubes 5D1 to 5D2 which are connectable to a tank 4 storing a culture solution. The plurality of tubes 5A1 to 5A5, 5B1 to 5B3, 5C1 to 5C2, and 5D1 to 5D2 are provided correspondingly to at least one of the plurality of cassettes 21 to 25, 31 to 34, and 41 to 43 constituting the sub-blocks 20, 30, and 40.

### <Pump connection tube>

A plurality of (in the example of FIG. 4, five for one pump 3) pump connection tubes 5A1 to 5A5 are provided. The pump connection tubes 5A1 to 5A5 include a first pump connection tube 5A1 into which a culture solution sent from the pump 3 flows, a second pump connection tube 5A2 disposed on a downstream side of the first pump connection tube 5A1 in a direction W in which a culture solution flows inside the pipe 5, a third pump connection tube 5A3 disposed on a downstream side of the second pump connection tube 5A2, a fourth pump connection tube 5A4 disposed on a downstream side of the third pump connection tube 5A3, and a fifth pump connection tube 5A5 disposed on a downstream side of the fourth pump connection tube 5A4. The number of tubes, which constitute the pump connection tubes 5A1 to 5A5, to be installed is not limited to that described above and can be changed in accordance with the required specifications.

In the cross-sectional view of FIG. 4, the first pump connection tube 5A1 extends from a positive Z end to the positive Z side of the pump 3 and then extends to the positive X side toward a part in the vicinity of the positive Z end of a negative X end of the first pump cassette 21. In the cross-sectional view of FIG. 4, the second pump connection tube 5A2 extends to the positive X side toward a positive X end of the second pump cassette 22 from a part in the vicinity of the positive X end of the first pump connection tube 5A1.

As illustrated in FIG. 3, a plurality of (in the example of FIG. 3, six for one pump 3) second pump connection tubes 5A2 are provided. In the top view of FIG. 3, the second pump connection tube 5A2 of the six second pump connection tubes disposed on the outermost side in the Y direction extends from the pump 3 side to the positive X side and then extends toward the outward side in the Y direction of the first pump cassette 21. Thereafter, it extends to the positive X side. In the top view of FIG. 3, the second pump connection tube 5A2 of the six second pump connection tubes disposed second to the outermost side in the Y direction extends to the positive X side from a part on the inward side in the Y direction from the second pump connection tube 5A2 on the outermost side in the pump 3 and then extends obliquely toward the outward side of the second pump cassette 22 in the Y direction. Thereafter, it extends to the positive X side. In the top view of FIG. 3, the second pump connection tube 5A2 of the six second pump connection tubes disposed the innermost side in the Y direction extends to the positive X side from a part on the inward side in the Y direction from the second pump connection tube 5A2 disposed second to the outermost side in the pump 3 and then extends toward the outward side of the second pump cassette 22 in the Y direction in a manner of being gently inclined from the second pump connection tube 5A2 disposed second to the outermost side. Thereafter, it extends to the positive X side.

In the cross-sectional view of FIG. 4, the third pump connection tube 5A3 extends to the negative Z side inside the fifth pump cassette 25 from a part in the vicinity of the positive Z end of the fifth pump cassette 25. In the cross-sectional view of FIG. 4, the fourth pump connection tube 5A4 extends to the negative X side inside the first pump cassette 21 from a part in the vicinity of a negative Z end of the positive X end of the first pump cassette 21. In the cross-sectional view of FIG. 4, the fifth pump connection tube 5A5 extends to the negative X side from a part in the vicinity of the negative Z end of the negative X end of the first pump cassette 21 and then extends to the positive Z side toward the negative Z end of the pump 3.

### <Relay tube>

A plurality of (three in the example of FIG. 4) relay tubes 5B1 to 5B3 are provided. The relay tubes 5B1 to 5B3 include a first relay tube 5B1 disposed on a downstream side of the second pump connection tube 5A2 in the direction W in which a culture solution flows inside the pipe 5, a second relay tube 5B2 disposed on a downstream side of the first relay tube 5B1, and a third relay tube 5B3 disposed on a downstream side of the second relay tube 5B2. The number of tubes, which constitute the relay tubes 5B1 to 5B3, to be installed is not limited to that described above and can be changed in accordance with the required specifications.

As illustrated in FIG. 3, a plurality of (for example, in the present embodiment, six correspondingly to the second pump connection tubes 5A2) first relay tubes 5B1 are provided. In the top view of FIG. 3, the first pump connection tube 5A1 extends in the X direction inside the tank block 30.

In the cross-sectional view of FIG. 4, the first relay tube 5B1 extends to the positive X side from the negative X side of the positive Z end of the second tank cassette 32. In the cross-sectional view of FIG. 4, the second relay tube 5B2 extends to the negative Z side toward one tank 4 from a downstream side of the positive X end of the first relay tube 5B1. In the cross-sectional view of FIG. 4, the third relay tube 5B3 extends to the positive Z side toward a part on the negative X side of the negative X end of the first relay tube 5B1 at the positive Z end of the second tank cassette 32 from the same position as the downstream end of the second relay tube 5B2 in the Z direction.

### <Coupling tube>

The coupling tubes 5C1 to 5C2 include a first coupling tube 5C1 disposed between the first pump connection tube 5A1 and the first relay tube 5B1 in the direction W in which a culture solution flows inside the pipe 5, and a second coupling tube 5C2 disposed between the third relay tube 5B3 and the third pump connection tube 5A3. For example, a plurality of first coupling tubes 5C1 may be provided correspondingly to the first pump connection tube 5A1. The number of tubes, which constitute the coupling tubes 5C1 to 5C2, to be installed is not limited to that described above and can be changed in accordance with the required specifications.

In the cross-sectional view of FIG. 4, the first coupling tube 5C1 extends in the X direction on the positive Z end side of the second couple cassette 42. In the cross-sectional view of FIG. 4, the second coupling tube 5C2 extends in the X direction on the positive Z end side of the third couple cassette 43. The length of the second coupling tube 5C2 in the X direction is shorter than the length of the first coupling tube 5C1 in the X direction.

### <Tank connection tube>

The tank connection tubes 5D1 to 5D2 include a first tank connection tube 5D1 disposed on a downstream side of the second relay tube 5B2 in the direction W in which a culture solution flows inside the pipe 5, and a second tank connection tube 5D2 disposed between the first tank connection tube 5D1 and the third relay tube 5B3. For example, a plurality of first tank connection tubes 5D1 may be provided correspondingly for the one tank 4. For example, a plurality of second tank connection tubes 5D2 may be provided correspondingly for the other tank 4 (a tank 4 disposed on the negative X side of the one tank 4). The number of tubes, which constitute the tank connection tubes 5D1 to 5D2, to be installed is not limited to that described above and can be changed in accordance with the required specifications.

In the cross-sectional view of FIG. 4, the first tank connection tube 5D1 extends to the negative Z side toward the inside of the one tank 4 from a part in the vicinity of the negative Z end of the second relay tube 5B2. In the cross-sectional view of FIG. 4, the second tank connection tube 5D2 extends to the positive Z side toward the negative X side inside the third tank cassette 33 from the inside of the other tank 4.

### <Joint member>

The cell culture device 1 may include joint members 6 connected in a manner of being attachable and detachable with respect to the tubes 5A1 to 5A5, 5B1 to 5B3, and 5C1 to 5C2. The joint members 6 are formed of a material with lower drug adsorption compared to the block 2. For example, when the block 2 is formed of an ABS resin, it is preferable that the joint members 6 be formed of a polyether ether ketone resin (PEEK resin), a fluororesin (for example, polytetrafluoroethylene or the like), a fluorine rubber, or the like.

The joint members 6 are provided at parts where the plurality of sub-blocks 20, 30, and 40 are connected to each other. The joint members 6 are provided at parts where the plurality of cassettes 21 to 25, 31 to 34, and 41 to 43 constituting the sub-blocks 20, 30, and 40 are connected to each other. A plurality of joint members 6 are provided. The plurality of joint members 6 include joint tubes (an example of tubes). The plurality of joint members 6 are formed to have various shapes, such as a shape extending in one direction, an L-shape, and a block shape.

The plurality of joint members 6 include a plurality of (five in the example of FIG. 4) pump joint tubes 6A1 to 6A5 connected in a manner of being attachable and detachable with respect to the pump connection tubes 5A1 to 5A5, a plurality of (four in the example of FIG. 4) relay joint tubes 6B1 to 6B4 connected in a manner of being attachable and detachable with respect to the relay tubes 5B1 to 5B3, and a plurality of (four in the example of FIG. 4) coupling joint tubes 6C1 to 6C4 connected in a manner of being attachable and detachable with respect to the coupling tubes 5C1 to 5C2.

### <Pump joint tube>

In the cross-sectional view of FIG. 4, the five pump joint tubes 6A1 to 6A5 include a first pump joint tube 6A1 connected in a manner of being attachable and detachable with respect to the positive Z end side of the first pump cassette 21, a second pump joint tube 6A2 connected in a manner of being attachable and detachable with respect to the fourth pump cassette 24 of the second pump cassette 22, a third pump joint tube 6A3 connected in a manner of being attachable and detachable with respect to the fourth pump cassette 24 and the fifth pump cassette 25, a fourth pump joint tube 6A4 connected in a manner of being attachable and detachable with respect to the second pump cassette 22 to the fifth pump cassette 25, and a fifth pump joint tube 6A5 connected in a manner of being attachable and detachable with respect to the negative Z end side of the first pump cassette 21. The number of tubes, which constitute the pump joint tubes 6A1 to 6A5, to be installed is not limited to that described above and can be changed in accordance with the required specifications.

In the cross-sectional view of FIG. 4, the first pump joint tube 6A1 extends in the X direction. In the cross-sectional view of FIG. 4, the first pump joint tube 6A1 has the negative X end connected to the positive X end of the first pump connection tube 5A1, and the positive X end connected to the negative X end of the second pump connection tube 5A2.

In the cross-sectional view of FIG. 4, the second pump joint tube 6A2 is formed to have an L-shape. In the cross-sectional view of FIG. 4, the second pump joint tube 6A2 has the negative X end connected to the positive X end of the second pump connection tube 5A2, and the positive Z end disposed on the positive Z end side of the third pump cassette 23. The second joint tube may include a plurality of joint tubes connected in a manner of being attachable to and detachable from each other.

In the cross-sectional view of FIG. 4, the third pump joint tube 6A3 extends in the Z direction. In the cross-sectional view of FIG. 4, the third pump joint tube 6A3 has the negative Z end connected to the positive Z end of the third pump connection tube 5A3, and the positive Z end disposed on the positive Z end side of the fifth pump cassette 25.

In the cross-sectional view of FIG. 4, the fourth pump joint tube 6A4 is formed to have an L-shape. In the cross-sectional view of FIG. 4, the length of the fourth pump joint tube 6A4 in the X direction is longer than the length of the second pump joint tube 6A2 in the X direction. In the cross-sectional view of FIG. 4, the fourth pump joint tube 6A4 has the positive Z end connected to the negative Z end of the third pump connection tube SA3, and the negative X end connected to the positive X end of the fourth pump connection tube 5A4. The fourth pump joint tube 6A4 may include a plurality of joint tubes connected in a manner of being attachable to and detachable from each other.

In the cross-sectional view of FIG. 4, the fifth pump joint tube 6A5 extends in the X direction parallel to the first pump joint tube 6A1. In the cross-sectional view of FIG. 4, the fifth pump joint tube 6A5 has the positive X end connected to the negative X end of the fourth pump connection tube 5A4, and the negative X end connected to the positive X end of the fifth pump connection tube 5A5.

### <Relay joint tube>

In the cross-sectional view of FIG. 4, the four relay joint tubes 6B1 to 6B4 include a first relay joint tube 6B1 connected in a manner of being attachable and detachable with respect to the negative X end side of the first tank cassette 31 and the second tank cassette 32, a second relay joint tube 6B2 connected in a manner of being attachable and detachable with respect to the positive X end side of the first tank cassette 31 to the third tank cassette 33, a third relay joint tube 6B3 connected in a manner of being attachable and detachable with respect to the negative X end side of the second tank cassette 32 and the third tank cassette 33, and a fourth relay joint tube 6B4 connected in a manner of being attachable and detachable with respect to a part on the negative X side of the first relay joint tube 6B1 on the negative X end side of the first tank cassette 31 and the second tank cassette 32. The number of tubes, which constitute the relay joint tubes 6B1 to 6B4, to be installed is not limited to that described above and can be changed in accordance with the required specifications.

In the cross-sectional view of FIG. 4, the first relay joint tube 6B1 is formed to have an L-shape. In the cross-sectional view of FIG. 4, the first relay joint tube 6B1 has the positive Z end disposed on the positive Z end side of the first tank cassette 31, and the positive X end connected to the negative X end of the first relay tube 5B1.

In the cross-sectional view of FIG. 4, the second relay joint tube 6B2 is formed to have a block shape extending in the Z direction. In the cross-sectional view of FIG. 4, the second relay joint tube 6B2 has the negative X end connected to the positive X end of the first relay tube 5B1, and the negative Z end disposed on the positive X end side on the negative Z end side of the third tank cassette 33 (in other words, the negative Z end connected to the positive Z end of the first tank connection tube 5D1). The second relay joint tube 6B2 may include a plurality of joint tubes connected in a manner of being attachable to and detachable from each other.

In the cross-sectional view of FIG. 4, the third relay joint tube 6B3 extends in the Z direction. In the cross-sectional view of FIG. 4, the third relay joint tube 6B3 has the negative Z end connected to the positive Z end of the second tank connection tube 5D2, and the positive Z end connected to the negative Z end of the third relay tube 5B3.

In the cross-sectional view of FIG. 4, the fourth relay joint tube 6B4 extends in the Z direction. In the cross-sectional view of FIG. 4, the fourth relay joint tube 6B4 has the negative Z end connected to the positive Z end of the third relay tube 5B3, and the positive Z end disposed on the negative X side of the first relay joint tube 6B1 on the positive Z end side of the first tank cassette 31.

### <Coupling joint tube>

In the cross-sectional view of FIG. 4, the four coupling joint tubes 6C1 to 6C4 include a first coupling joint tube 6C1 connected in a manner of being attachable and detachable with respect to the negative X end side of the first couple cassette 41 to the third couple cassette 43, a second coupling joint tube 6C2 connected in a manner of being attachable and detachable with respect to the positive X end side of the first couple cassette 41 to the third couple cassette 43, a third coupling joint tube 6C3 connected in a manner of being attachable and detachable with respect to the positive X end side of the second couple cassette 42 and the third couple cassette 43, and a fourth coupling joint tube 6C4 connected in a manner of being attachable and detachable with respect to the negative X end side of the second couple cassette 42 and the third couple cassette 43. The number of tubes, which constitute the coupling joint tubes 6C1 to 6C4, to be installed is not limited to that described above and can be changed in accordance with the required specifications.

In the cross-sectional view of FIG. 4, the first coupling joint tube 6C1 is formed to have an L-shape. In the cross-sectional view of FIG. 4, the first coupling joint tube 6C1 has the negative Z end connected to the positive Z end of the second pump joint tube 6A2, and the positive X end connected to the negative X end of the first coupling tube 5C1. The first coupling joint tube 6C1 may include a plurality of joint tubes connected in a manner of being attachable to and detachable from each other.

In the cross-sectional view of FIG. 4, the second coupling joint tube 6C2 is formed to have an L-shape. In the cross-sectional view of FIG. 4, the second coupling joint tube 6C2 has the negative X end connected to the positive X end of the first coupling tube 5C1, and the negative Z end connected to the positive Z end of the first relay joint tube 6B1. The second coupling j oint tube 6C2 may include a plurality of joint tubes connected in a manner of being attachable to and detachable from each other.

In the cross-sectional view of FIG. 4, the third coupling joint tube 6C3 is formed to have an L-shape. In the cross-sectional view of FIG. 4, the length of the third coupling joint tube 6C3 in the Z direction is shorter than the length of the second coupling joint tube 6C2 in the Z direction. In the cross-sectional view of FIG. 4, the third coupling joint tube 6C3 has the negative Z end connected to the positive Z end of the third relay tube 5B3, and the negative X end connected to the positive X end of the second coupling tube 5C2.

In the cross-sectional view of FIG. 4, the fourth coupling joint tube 6C4 is formed to have an L-shape. In the cross-sectional view of FIG. 4, the length of the fourth coupling joint tube 6C4 in the Z direction is shorter than the length of the third coupling joint tube 6C3 in the Z direction. In the cross-sectional view of FIG. 4, the fourth coupling joint tube 6C4 has the positive X end connected to the negative X end of the second coupling tube 5C2, and the negative Z end connected to the positive Z end of the third pump joint tube 6A3.

### <Guide portion>

The guide portions 10 are provided at parts corresponding to the pipe 5 and the joint members 6 in the block 2. In the cross-sectional view of FIG. 4, the guide portions 10 include the pump-side guide portions 10A1 to 10A8 provided in the pump block 20 (refer to FIG. 5), the relay guide portions 10B1 to 10B6 provided in the tank block 30 (refer to FIG. 6), and the coupling guide portions 10C1 to 10C6 provided in the coupling block 40 (refer to FIG. 7).

### <Pump-side guide portion>

As illustrated in FIG. 5, the pump-side guide portions 10A1 to 10A8 include a first pump-side guide portion 10A1 in which the first pump joint tube 6A1 is attachable and detachable, a second pump-side guide portion 10A2 in which the second pump connection tube 5A2 is attachable and detachable, a third pump-side guide portion 10A3 in which the second pump joint tube 6A2 is attachable and detachable, a fourth pump-side guide portion 10A4 in which the third pump joint tube 6A3 is attachable and detachable, a fifth pump-side guide portion 10A5 in which the third pump connection tube 5A3 is attachable and detachable, a sixth pump-side guide portion 10A6 in which the fourth pump joint tube 6A4 is attachable and detachable, a seventh pump-side guide portion 10A7 in which the fourth pump connection tube 5A4 is attachable and detachable, and an eighth pump-side guide portion 10A8 in which the fifth pump joint tube 6A5 is attachable and detachable. The number of guide portions, which constitute the pump-side guide portions 10A1 to 10A8, to be installed is not limited to that described above and can be changed in accordance with the required specifications.

The pump-side guide portions 10A1 to 10A8 include the recessed portion 11 guiding the pump joint tubes 6A1 to 6A5 or the pump connection tubes 5A1 to 5A5 along surfaces of the pump cassettes 21 to 25, and the hole portion 12 guiding the pump joint tubes 6A1 to 6A5 or the pump connection tubes 5A1 to 5A5 through the inside of the pump cassettes 21 to 25.

As illustrated in FIG. 1, the first pump-side guide portion 10A1 includes a penetration hole extending in the Y direction when viewed in the X direction.

The second pump-side guide portion 10A2 includes the recessed portion 11 depressed from a positive Z surface of the first pump cassette 21 to the negative Z side and having a rectangular shape when viewed in the X direction.

The third pump-side guide portion 10A3 includes a plurality of (six disposed with a gap therebetween in the Y direction in the example of FIG. 1) penetration holes having a circular shape when viewed in the X direction. In the cross-sectional view of FIG. 5, the third pump-side guide portion 10A3 includes an L-shaped penetration hole penetrating the inside of the second pump cassette 22 to the fourth pump cassette 24 on the positive Z end side.

In the cross-sectional view of FIG. 5, the fourth pump-side guide portion 10A4 includes an opening portion opening on the positive Z end side of the fourth pump cassette 24 and the fifth pump cassette 25 (in other words, an opening portion including a groove extending in the Z direction along a boundary surface between the fourth pump cassette 24 and the fifth pump cassette 25).

In the cross-sectional view of FIG. 5, the fifth pump-side guide portion 10A5 includes a groove extending in the Z direction along a negative X end surface (an example of a surface) of the fifth pump cassette 25.

In the cross-sectional view of FIG. 5, the sixth pump-side guide portion 10A6 includes an L-shaped penetration hole penetrating the inside of the second pump cassette 22 to the fifth pump cassette 25 on the negative Z end side.

In the cross-sectional view of FIG. 5, the seventh pump-side guide portion 10A7 includes a penetration hole penetrating the inside of the first pump cassette 21 on the negative Z end side in the X direction.

As illustrated in FIG. 1, the eighth pump-side guide portion 10A8 includes a penetration hole extending in the Y direction parallel to the first pump-side guide portion 10A1 when viewed in the X direction.

### <Relay guide portion>

As illustrated in FIG. 6, the relay guide portions 10B1 to 10B6 include a first relay guide portion 10B1 in which the first relay joint tube 6B 1 is attachable and detachable, a second relay guide portion 10B2 in which the first relay tube 5B1 is attachable and detachable, a third relay guide portion 10B3 in which the second relay joint tube 6B2 is attachable and detachable, a fourth relay guide portion 10B4 in which the third relay joint tube 6B3 is attachable and detachable, a fifth relay guide portion 10B5 in which the third relay tube 5B3 is attachable and detachable, and a sixth relay guide portion 10B6 in which the fourth relay joint tube 6B4 is attachable and detachable. The number of guide portions, which constitute the relay guide portions 10B1 to 10B6, to be installed is not limited to that described above and can be changed in accordance with the required specifications.

The relay guide portions 10B1 to 10B6 include a recessed portion guiding the relay j oint tubes 6B1 to 6B4 or the relay tubes 5B1 to 5B3 along surfaces of the tank cassettes 31 to 33, and a hole portion guiding the relay joint tubes 6B1 to 6B4 or the relay tubes 5B1 to 5B3 through the inside of the tank cassettes 31 to 33.

In the cross-sectional view of FIG. 6, the first relay guide portion 10B1 includes an L-shaped opening portion opening the inside of the first tank cassette 31 and the second tank cassette 32 on the negative X end side.

In the cross-sectional view of FIG. 6, the second relay guide portion 10B2 includes a groove extending in the X direction along a positive Z end surface (an example of a surface) of the second tank cassette 32.

As illustrated in FIG. 1, the third relay guide portion 10B3 includes a rectangular opening portion depressed from the positive Z surface of the first tank cassette 31 to the negative Z side when viewed in the Z direction. In the cross-sectional view of FIG. 6, the third relay guide portion 10B3 includes a penetration hole penetrating the inside of the third tank cassette 33 on the positive X end side from the first tank cassette 31 in the Z direction.

In the cross-sectional view of FIG. 6, the fourth relay guide portion 10B4 includes an opening portion opening the inside of the second tank cassette 32 and the third tank cassette 33 on the negative X end side in the Z direction.

In the cross-sectional view of FIG. 6, the fifth relay guide portion 10B5 includes an opening portion opening the inside of the second tank cassette 32 on the negative X end side in the Z direction.

In the cross-sectional view of FIG. 4, the sixth relay guide portion 10B6 includes an opening portion opening on the negative X side of the first relay guide portion 10B1 in the Z direction inside the first tank cassette 31 and the second tank cassette 32 on the negative X end side.

### <Coupling guide portion>

As illustrated in FIG. 7, the coupling guide portions 10C1 to 10C6 include the first coupling guide portion 10C1 in which the first coupling joint tube 6C1 is attachable and detachable, the second coupling guide portion 10C2 in which the first coupling tube 5C1 is attachable and detachable, the third coupling guide portion 10C3 in which the second coupling joint tube 6C2 is attachable and detachable, the fourth coupling guide portion 10C4 in which the third coupling joint tube 6C3 is attachable and detachable, the fifth coupling guide portion 10C5 in which the second coupling tube 5C2 is attachable and detachable, and the sixth coupling guide portion 10C6 in which the fourth coupling joint tube 6C4 is attachable and detachable. The number of guide portions, which constitute the coupling guide portions 10C1 to 10C6, to be installed is not limited to that described above and can be changed in accordance with the required specifications.

The coupling guide portions 10C1 to 10C6 include a recessed portion guiding the coupling joint tubes 6C1 to 6C4 or the coupling tubes 5C1 to 5C2 along surfaces of the couple cassettes 41 to 43, and a hole portion guiding the coupling joint tubes 6C1 to 6C4 or the coupling tubes 5C1 to 5C2 through the inside of the couple cassettes 41 to 43.

In the cross-sectional view of FIG. 7, a first coupling guide portion 10C1 includes an L-shaped opening portion opening the inside of the first couple cassette 41 to the third couple cassette 43 on the negative X end side.

In the cross-sectional view of FIG. 7, a second coupling guide portion 10C2 includes a groove extending in the X direction along the positive Z end surface (an example of a surface) of the second couple cassette 42.

In the cross-sectional view of FIG. 7, a third coupling guide portion 10C3 includes an L-shaped opening portion opening the inside of the first couple cassette 41 to the third couple cassette 43 on the positive X end side.

In the cross-sectional view of FIG. 7, a fourth coupling guide portion 10C4 includes an L-shaped opening portion opening on the negative X side of the third coupling guide portion 10C3 inside the second couple cassette 42 and the third couple cassette 43 on the positive X end side.

In the cross-sectional view of FIG. 7, a fifth coupling guide portion 10C5 includes a groove extending in the X direction along the positive Z end surface (an example of a surface) of the third couple cassette 43.

In the cross-sectional view of FIG. 7, a sixth coupling guide portion 10C6 includes an L-shaped opening portion opening between the first coupling guide portion 10C1 and the fifth coupling guide portion 10C5 inside the second couple cassette 42 and the third couple cassette 43 on the negative X end side.

### <Supply cassette>

As illustrated in FIG. 6, the supply cassette 34 may be attached to the tank block 30. The supply cassette 34 has supply guide portions 10D1 to 10D2 (an example of guide portions) in which the tank connection tubes 5D1 and 5D2 (an example of supply tubes capable of supplying a culture solution) is attachable and detachable with respect to a culture chip 35 which is capable of storing a culture solution. The culture chip 35 has a flow channel for culturing cells.

The supply cassette 34 is connected in a manner of being attachable and detachable with respect to the third tank cassette 33. The supply cassette 34 is connected in a manner of being attachable and detachable with respect to an upper portion of the culture chip 35 (positive Z end portion). The supply cassette 34 has tank mounting portions 34a in which the tanks 4 can be mounted. A plurality of (two in the example of FIG. 6) tank mounting portions 34a are provided correspondingly to a plurality of (two in the example of FIG. 6) tanks 4 inside the supply cassette 34. The number of tanks 4 to be installed is not limited to that described above and can be changed in accordance with the required specifications.

The supply guide portions 10D1 to 10D2 include a first supply guide portion 10D1 in which one supply tube (first tank connection tube 5D1) is attachable and detachable, and a second supply guide portion 10D2 in which the other supply tube (second tank connection tube 5D2) is attachable and detachable. The number of guide portions, which constitute the supply guide portions 10D1 to 10D2, to be installed is not limited to that described above and can be changed in accordance with the required specifications.

The supply guide portions 10D1 to 10D2 include a hole portion guiding the tank connection tubes 5D1 and 5D2 through the inside of the supply cassette 34.

In the cross-sectional view of FIG. 6, the first supply guide portion 10D1 includes a penetration hole opening the inside on the positive X end side in the Z direction in a wall portion of the supply cassette 34 on the positive Z end side.

In the cross-sectional view of FIG. 6, the second supply guide portion 10D2 includes a penetration hole opening the inside on the negative X end side in the Z direction in the wall portion of the supply cassette 34 on the positive Z end side.

### <Pipe connection system>

As illustrated in FIG. 3, the cell culture device 1 has pipe connection systems 9A to 9F and 9G to 9L that are different from each other in accordance with the purpose of cell culture. The pipe connection systems 9A to 9F and 9G to 9L include first pipe connection systems 9A to 9F connected to one pump 3, and second pipe connection systems 9G to 9L connected to the other pump 3.

The first pipe connection systems 9A to 9F are disposed at a part of the block 2 on the negative Y side. For example, the first pipe connection systems 9A to 9F include the pipe 5 (in the example of FIG. 3, six tubes 9A to 9F extending from the pump 3 on the negative Y side) through which a first culture solution circulates.

The second pipe connection systems 9G to 9L are disposed at a part of the block 2 on a positive Y side. For example, the second pipe connection systems 9G to 9L include the pipe 5 (in the example of FIG. 3, six tubes 9G to 9L extending from the pump 3 on the positive Y side) through which a second culture solution circulates. For example, the second culture solution may be a liquid different from the first culture solution.

### <Flow of culture solution>

Hereinafter, a flow of a culture solution in one pipe connection system of the plurality of pipe connection systems 9A to 9F and 9G to 9L constituting the cell culture device 1 will be described.

As illustrated in FIG. 4, when the cell culture device 1 is in use, while the cassettes 21 to 25, 31 to 34, and 41 to 43 are coupled to each other, the sub-blocks 20, 30, and 40 are coupled to each other. In addition, while the pump 3 is mounted in the pump mounting portion 21a, the tank 4 is mounted in the tank mounting portion 34a. In addition, the tubes 5A1 to 5A5, 5B1 to 5B3, 5C1 to 5C2, 5D1 to 5D2, 6A1 to 6A5, 6B1 to 6A4, and 6C1 to 6C4 (the pipe 5 and the joint members 6) are attached to the guide portions 10 of the block 2. When the cell culture device 1 is in use, a coupling order of the cassettes 21 to 25, 31 to 34, and 41 to 43 and the sub-blocks 20, 30, and 40, a mounting order of the pumps 3 and the tank 4, and an attachment order of the tubes 5A1 to 5A5, 5B1 to 5B3, 5C1 to 5C2, 5D1 to 5D2, 6A1 to 6A5, 6B1 to 6A4, and 6C1 to 6C4 are not limited and can be changed as necessary.

In the example of FIG. 4, starting from the pump 3, the first pump connection tube 5A1, the first pump joint tube 6A1, the second pump connection tube 5A2, the second pump joint tube 6A2, the first coupling joint tube 6C1, the first coupling tube 5C1, the second coupling joint tube 6C2, the first relay joint tube 6B1, the first relay tube 5B1, the second relay joint tube 6B2, the second relay tube 5B2, the first tank connection tube 5D1, the one tank 4, and the culture chip 35 are sequentially connected.

In addition, starting from the culture chip 35, the other tank 4, the second tank connection tube 5D2, the third relay tube 5B3, the third relay joint tube 6B3, the third relay tube 5B3, the fourth relay joint tube 6B4, the third coupling joint tube 6C3, the second coupling tube 5C2, the fourth coupling joint tube 6C4, the third pump joint tube 6A3, the third pump connection tube 5A3, the fourth pump joint tube 6A4, the fourth pump connection tube 5A4, the fifth pump joint tube 6A5, the fifth pump connection tube 5A5, and the pump 3 are sequentially connected.

In such a connection state, when the pump 3 is driven, a culture solution sent from the pump 3 flows in an arrow W direction and flows into the culture chip 35 via the first pump connection tube 5A1, the first pump joint tube 6A1, the second pump connection tube 5A2, the second pump joint tube 6A2, the first coupling joint tube 6C1, the first coupling tube 5C1, the second coupling joint tube 6C2, the first relay joint tube 6B1, the first relay tube 5B1, the second relay j oint tube 6B2, the second relay tube 5B2, the first tank connection tube 5D1, and the one tank 4.

In addition, the culture solution inside the other tank 4 returns to the pump 3 via the second tank connection tube 5D2, the third relay tube 5B3, the third relay joint tube 6B3, the third relay tube 5B3, the fourth relay joint tube 6B4, the third coupling joint tube 6C3, the second coupling tube 5C2, the fourth coupling joint tube 6C4, the third pump joint tube 6A3, the third pump connection tube 5A3, the fourth pump joint tube 6A4, the fourth pump connection tube 5A4, the fifth pump joint tube 6A5, and the fifth pump connection tube 5A5.

The culture solution which has returned to the pump 3 flows in the arrow W direction passes through the path described above. That is, a circulation path for a culture solution can be formed by the tubes 5A1 to 5A5, 5B1 to 5B3, 5C1 to 5C2, 5D1 to 5D2, 6A1 to 6A5, 6B 1 to 6A4, and 6C1 to 6C4 (the pipe 5 and the joint members 6).

As necessary, a culture solution collection portion for collecting a culture solution may be provided in the middle of the circulation path.

### <Operational effects>

As above, the cell culture device 1 of the present embodiment includes the block 2 that has the guide portion 10 in which the pipe 5 allowing a culture solution to circulate therein is attachable and detachable. The guide portion 10 includes the recessed portion 11 that guides the pipe 5 along a surface of the block 2, and the hole portion 12 that guides the pipe 5 through the inside of the block 2.

According to the present embodiment, since the pipe 5 through which a culture solution circulates and the block 2 are segregated, even when the pipe 5 is contaminated by a culture solution, contamination of the block 2 can be avoided and the block 2 can be reused in terms of cost. Furthermore, when the pipe 5 is contaminated, the contamination can be curbed by removing the pipe 5 from the guide portion 10 of the block 2 and attaching an uncontaminated pipe 5 to the guide portion 10. Therefore, costs can be reduced and contamination can be curbed. Furthermore, cytotoxicity caused by a culture solution remaining inside the pipe 5 can be reduced. Furthermore, when the pipe 5 is attached to the guide portion 10 of the block 2, since the pipe 5 can be guided by at least one of the recessed portion 11 of the block 2 and the inside of the block 2, a situation in which the pipe 5 is mixed up in a mess can be curbed. Therefore, occurrence of erroneous connection of the pipe 5 or leakage of a culture solution can be curbed and unnecessary use of a culture solution due to an excessively long pipe 5 can be curbed.

In the present embodiment, the block 2 includes a plurality of first pump cassettes 21 that are coupled in a manner of being attachable to and detachable from each other. The plurality of first pump cassettes 21 have pipe connection systems 9A to 9F and 9G to 9L that are different from each other in accordance with the purpose of cell culture.

According to this constitution, since the pipe connection systems 9A to 9F and 9G to 9L can be varied in accordance with the purpose of cell culture, an optimal device for cell culture can be provided.

In the present embodiment, the block 2 includes the supply cassette 34 that has the supply guide portions 10D1 to 10D2 in which the supply tubes 5D1 and 5D2 capable of supplying a culture solution is attachable and detachable with respect to the culture chip 35 capable of storing a culture solution. The supply cassette 34 is connected in a manner of being attachable and detachable with respect to the upper portion of the culture chip 35.

According to this constitution, since the supply cassette 34 is connectable to the upper portion of the culture chip 35 when the device is in use and the supply cassette 34 can be detached when the device is not in use, it is suitable for managing the supply cassette 34. Furthermore, compared to a constitution in which the supply cassette 34 is connected to a lower portion of the culture chip 35, connection work can be made efficient.

In the present embodiment, the block 2 is formed of a resin.

According to this constitution, the weight can be reduced compared to when the block 2 is formed of a metal.

In the present embodiment, the pipe 5 is formed of a material with lower drug adsorption compared to the block 2.

According to this constitution, since a situation in which a drug remains inside the pipe 5 can be curbed, an optimal device for evaluation of a drug can be provided.

In the present embodiment, the block 2 includes the plurality of sub-blocks 20, 30, and 40 that are segregated in a manner of being attachable to and detachable from each other.

According to this constitution, the block 2 can be made compact in units of the plurality of sub-blocks 20, 30, and 40 constituting it. Furthermore, for instance, even when the block 2 is partially contaminated, since only the contaminated one of the plurality of sub-blocks 20, 30, and 40 constituting the block 2 can be discarded and the remainder can be reused, it is suitable for reducing costs and curbing contamination.

In the present embodiment, the pipe 5 includes the plurality of tubes 5A1 to 5A5, 5B1 to 5B3, 5C1 to 5C2, and 5D1 to 5D2 provided correspondingly to the plurality of sub-blocks 20, 30, and 40.

According to this constitution, the pipe 5 can be made compact in units of the plurality of tubes 5A1 to 5A5, 5B1 to 5B3, 5C1 to 5C2, and 5D1 to 5D2 constituting it. Furthermore, even when the pipe 5 is partially contaminated, since only the contaminated one of the plurality of tubes 5A1 to 5A5, 5B1 to 5B3, 5C1 to 5C2, and 5D1 to 5D2 constituting the pipe 5 can be discarded and the remainder can be reused, it is suitable for reducing costs and curbing contamination.

In the present embodiment, the plurality of tubes SA1 to 5A5, 5B1 to 5B3, 5C1 to 5C2, and 5D1 to 5D2 include the pump connection tubes 5A1 to 5A5 that are connectable to the pump 3, and the relay tubes 5B1 to 5B3 that relay the pipe. The plurality of sub-blocks 20, 30, and 40 include the pump block 20 that has the pump-side guide portions 10A1 to 10A8 in which the pump connection tubes SA1 to 5A5 is attachable and detachable, and the tank block 30 that has the relay guide portions 10B1 to 10B6 which are connected in a manner of being attachable and detachable with respect to the pump block 20 and in which the relay tubes 5B1 to 5B3 is attachable and detachable.

According to this constitution, even when one of the pump connection tubes SA1 to 5A5 or the relay tubes 5B1 to 5B3 is contaminated, only the contaminated one can be discarded and the remainder can be reused. Furthermore, for instance, even when one of the pump block 20 and the tank block 30 is contaminated only the contaminated one can be discarded and the remainder can be reused.

In the present embodiment, the pump block 20 has the pump mounting portion 21a in which the pump 3 can be mounted. The supply cassette 34 has the tank mounting portion 34a in which the tank 4 can be mounted.

According to this constitution, the tank 4 is mounted in the tank mounting portion 34a while the pump 3 is mounted in the pump mounting portion 21a when the device is in use, and the pump 3 and the tank 4 can be detached when the device is not in use. Therefore, it is suitable for managing the pump 3 and the tank 4.

In the present embodiment, the plurality of tubes 5A1 to 5A5, 5B1 to 5B3, 5C1 to 5C2, and 5D1 to 5D2 include the coupling tubes 5C1 to 5C2 that can couple the pump connection tubes SA1 to 5A5 and the relay tubes 5B1 to 5B3. The plurality of sub-blocks 20, 30, and 40 include the coupling block 40 that has the coupling guide portions 10C1 to 10C6 in which the coupling tubes 5C1 to 5C2 is attachable and detachable. The coupling block 40 may be connected in a manner of being attachable and detachable with respect to the pump block 20 and the tank block 30.

According to this constitution, even when any of the pump connection tubes 5A1 to 5A5, the coupling tubes 5C1 to 5C2, and the relay tubes 5B1 to 5B3 is contaminated, only the contaminated one can be discarded and the remainder can be reused. Furthermore, for instance, even when any of the pump block 20, the coupling block 40, and the tank block 30 is contaminated, only the contaminated one can be discarded and the remainder can be reused. Furthermore, since the pump connection tubes SA1 to 5A5 and the relay tubes 5B1 to 5B3 can be directly connected or can be connected via the coupling tubes 5C1 to 5C2, the degree of freedom in layout of the pipe 5 is improved. Furthermore, the pump block 20 and the tank block 30 can be directly connected or can be connected via the coupling block 40, the degree of freedom in layout of the block 2 is improved.

In the present embodiment, the plurality of sub-blocks 20, 30, and 40 include the plurality of cassettes 21 to 25, 31 to 34, and 41 to 43 that are connected in a manner of being attachable to and detachable from each other. The pipe 5 includes the plurality of tubes 5A1 to 5A5, 5B1 to 5B3, 5C1 to 5C2, and 5D1 to 5D2 that are provided correspondingly to at least one of the plurality of cassettes 21 to 25, 31 to 34, and 41 to 43.

According to this constitution, the sub-blocks 20, 30, and 40 can be made compact in units of the plurality of cassettes 21 to 25, 31 to 34, and 41 to 43 constituting them. Furthermore, for instance, even when the sub-blocks 20, 30, and 40 are partially contaminated, only the contaminated one of the plurality of cassettes 21 to 25, 31 to 34, and 41 to 43 constituting the sub-blocks 20, 30, and 40 can be discarded and the remainder can be reused. Furthermore, when the plurality of tubes SA1 to 5A5, 5B1 to 5B3, 5C1 to 5C2, and 5D1 to 5D2 are provided correspondingly to the plurality of cassettes 21 to 25, 31 to 34, and 41 to 43, the pipe 5 can be made compact in units of the plurality of tubes 5A1 to 5A5, 5B1 to 5B3, 5C1 to 5C2, and 5D1 to 5D2 constituting it. Furthermore, even when the pipe 5 is partially contaminated, only the contaminated one of the plurality of tubes 5A1 to 5A5, 5B1 to 5B3, 5C1 to 5C2, and 5D1 to 5D2 constituting the pipe 5 can be discarded and the remainder can be reused.

In the present embodiment, the cell culture device 1 includes the joint member 6 that is provided at a part where the plurality of sub-blocks 20, 30, and 40 are connected to each other and is connected in a manner of being attachable and detachable with respect to the tubes 5A1 to 5A5, 5B1 to 5B3, 5C1 to 5C2, and 5D1 to 5D2.

According to this constitution, since the tubes 5A1 to 5A5, 5B1 to 5B3, 5C1 to 5C2, and 5D1 to 5D2 can be easily attached and detached using the joint member 6, erroneous connection can be reduced and connection work can be made efficient.

### <Modification examples>

All the shapes, combinations, and the like of constituent members indicated in the examples described above are merely examples and can be variously changed based on design requirements or the like.

In the foregoing embodiment, the cell culture device includes the block that has the guide portion in which the pipe allowing a culture solution to circulate therein is attachable and detachable. The guide portion includes the recessed portion that guides the pipe along the surface of the block, and the hole portion that guides the pipe through the inside of the block. However, it is not limited to this. For example, the guide portion may have only the recessed portion that guides the pipe along the surface of the block or may have only the hole portion that guides the pipe through the inside of the block. For example, the guide portion need only include at least one of the recessed portion that guides the pipe along the surface of the block, and the hole portion that guides the pipe through the inside of the block. For example, the shape of the guide portion can be changed in accordance with the required specifications.

In the foregoing embodiment, the block includes the plurality of first pump cassettes that are coupled in a manner of being attachable to and detachable from each other. The plurality of first pump cassettes have the pipe connection systems that are different from each other in accordance with the purpose of cell culture. However, it is not limited to this. For example, the plurality of first pump cassettes may have the pipe connection systems that are the same as each other in accordance with the purpose of cell culture. For example, the shape of the pipe connection systems provided in the plurality of coupling blocks can be changed in accordance with the required specifications.

In the foregoing embodiment, the block includes the supply cassette that has the supply guide portion in which the supply tube capable of supplying a culture solution is attachable and detachable with respect to the culture chip which is capable of storing a culture solution. The supply cassette is connected in a manner of being attachable and detachable with respect to the upper portion of the culture chip. However, it is not limited to this. For example, the supply cassette may be connected to the lower portion of the culture chip. For example, a connection form of the supply cassette with respect to the culture chip can be changed in accordance with the required specifications.

In the foregoing embodiment, the block is formed of a resin. However, it is not limited to this.

For example, the block may be formed of a metal. For example, the material of the block can be changed in accordance with the required specifications.

In the foregoing embodiment, the pipe is formed of a material with lower drug adsorption compared to the block. However, it is not limited to this. For example, the pipe may be formed of a material with higher drug adsorption compared to the block. For example, the material of the pipe can be changed in accordance with the required specifications.

In the foregoing embodiment, the block includes the plurality of sub-blocks that are segregated in a manner of being attachable to and detachable from each other. However, it is not limited to this. For example, the block may not include the plurality of sub-blocks. For example, the block may be constituted of a single block. For example, the shape of the block can be changed in accordance with the required specifications.

In the foregoing embodiment, the pipe includes the plurality of tubes that are provided correspondingly to the plurality of sub-blocks. However, it is not limited to this. For example, the plurality of tubes may not be provided correspondingly to the plurality of sub-blocks. For example, the shape of the pipe can be changed in accordance with the required specifications.

In the foregoing embodiment, the plurality of tubes include the pump connection tube that is connectable to the pump, and the relay tube for relaying the pipe. The plurality of sub-blocks include the pump block that has the pump-side guide portion in which the pump connection tube is attachable and detachable, and tank block that has the relay guide portion which is connected in a manner of being attachable and detachable with respect to the pump block and in which the relay tube is attachable and detachable. However, it is not limited to this. For example, the plurality of tubes may include the tank connection tube that is connectable to the tank. The plurality of sub-blocks may include the tank block that has the tank-side guide portion which is connected in a manner of being attachable and detachable with respect to the pump block and in which the tank connection tube is attachable and detachable. For example, the plurality of sub-blocks may not include the pump block and the tank block. For example, the shape of the sub-block can be changed in accordance with the required specifications.

In the foregoing embodiment, the pump block has the pump mounting portion in which the pump can be mounted. The supply cassette has the tank mounting portion in which the tank can be mounted. However, it is not limited to this. For example, the pump block may not have the pump mounting portion. For example, the supply cassette may not have the tank mounting portion. For example, forms of the pump block and the supply cassette can be changed in accordance with the required specifications.

In the foregoing embodiment, the plurality of tubes include the coupling tube that can couple the pump connection tube and the relay tube. The plurality of sub-blocks include the coupling block that has the coupling guide portion in which the coupling tube is attachable and detachable. The coupling block is connected in a manner of being attachable and detachable with respect to the pump block and the tank block. However, it is not limited to this. For example, the plurality of tubes may include the coupling tube that can couple the pump connection tube and the tank connection tube. The plurality of sub-blocks may include the coupling block that has the coupling guide portion in which the coupling tube is attachable and detachable. The coupling block may be connected in a manner of being attachable and detachable with respect to the pump block and the tank block. For example, the coupling block may not be connected to the pump block and the tank block. For example, the pump block and the tank block may be connected in a manner of being attachable to and detachable from each other without the coupling block therebetween. For example, a connection form of each of the blocks can be changed in accordance with the required specifications.

In the foregoing embodiment, the plurality of sub-blocks include the plurality of cassettes that are connected in a manner of being attachable to and detachable from each other. The pipe includes the plurality of tubes that are provided correspondingly to at least one of the plurality of cassettes. However, it is not limited to this. For example, the plurality of tubes may not be provided correspondingly to at least one of the plurality of cassettes. For example, the shape of the pipe can be changed in accordance with the required specifications.

In the foregoing embodiment, the cell culture device includes the joint member that is provided at a part where the plurality of sub-blocks are connected to each other and is connected in a manner of being attachable and detachable with respect to the tubes. However, it is not limited to this. For example, the cell culture device may not include the joint member. For example, the tubes may be connected without the joint member therebetween. For example, a connection form of the tubes can be changed in accordance with the required specifications.

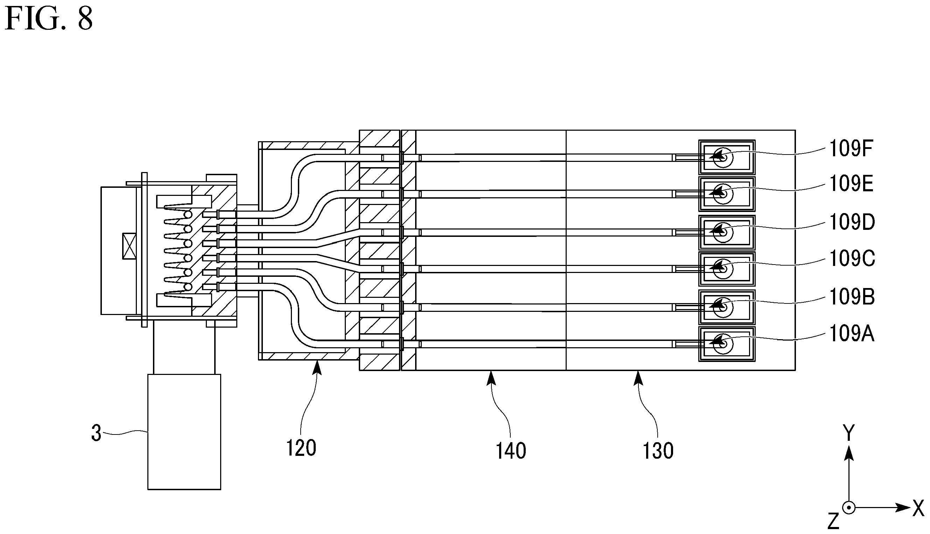

In the foregoing embodiment, the coupling block is disposed in the upper portion (a part on the positive Z side) of the pump block and the tank block. However, it is not limited to this. For example, as illustrated in FIG. 8, a coupling block 140 may be disposed between a pump block 120 and a tank block 130 in the X direction. For example, a disposition form of the coupling block can be changed in accordance with the required specifications.

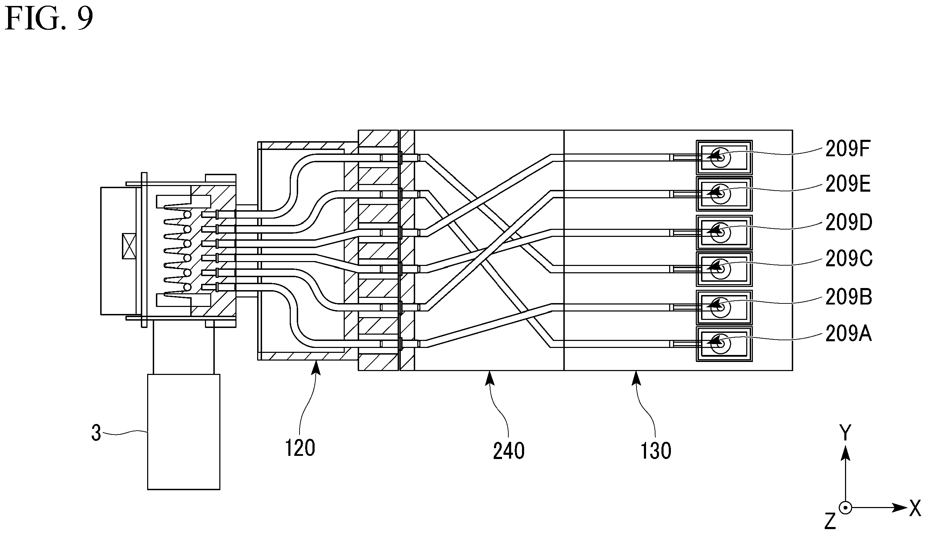

In the foregoing embodiment, the plurality of first pump cassettes have the pipe connection systems that are different from each other in accordance with the purpose of cell culture. However, it is not limited to this. For example, as illustrated in FIGS. 8 and 9, a plurality of coupling blocks 140 and 240 may have pipe connection systems 109A to 109F and 209A to 209F different from each other in accordance with the purpose of cell culture. For example, the pipe connection systems 109A to 109F and 209A to 209F may include the first pipe connection systems 109A to 109F that are provided in the first coupling block 140 (refer to FIG. 8), and the second pipe connection systems 209A to 209F provided in the second coupling block 240.

For example, as illustrated in FIG. 8, when the first coupling block 140 is disposed between the pump block 120 and the tank block 130, a culture solution circulates through each of the tubes 109A to 109F guided by the guide portions of the pump block 120, the first coupling block 140, and the tank block 130.

On the other hand, as illustrated in FIG. 9, when the second coupling block 240 is disposed between the pump block 120 and the tank block 130, a culture solution circulates through each of the tubes 209A to 209F guided by the guide portions of the pump block 120, the second coupling block 240, and the tank block 130. For example, the second pipe connection systems 209A to 209F intersect each other inside the second coupling block 240 in a three-dimensional manner.

For example, the shape of the pipe connection system can be changed in accordance with the required specifications.

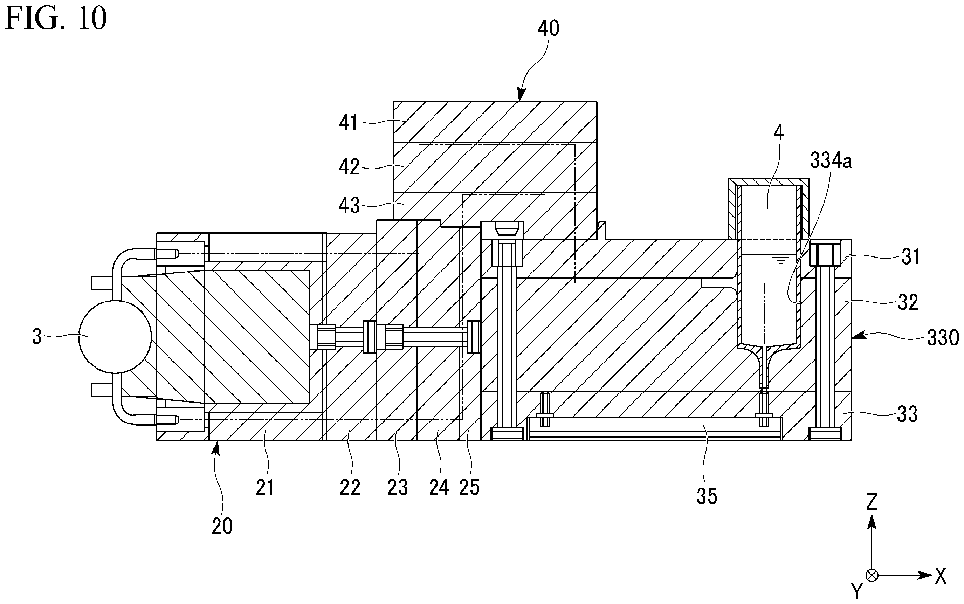

In the foregoing embodiment, the supply cassette has the tank mounting portion in which the tank can be mounted. However, it is not limited to this. For example, as illustrated in FIG. 10, a tank block 330 may have a tank mounting portion 334a in which the tank 4 can be mounted. In FIG. 10, the same reference signs are applied to constituents similar to those in the foregoing embodiment, and some constituent elements of the pipe are omitted.

For example, the supply cassette may not be attached to the tank block 330. For example, the culture chip 35 may be attached to the lower portion of the third tank cassette 33. For example, disposition forms of the supply cassette and the culture chip can be changed in accordance with the required specifications.

In the foregoing embodiment, the cell culture device culturing cells by circulating a cell culture liquid has been described. However, it is not limited to this. For example, the present invention may be applied to other fluid circuit devices such as a liquid supply device for circulating a liquid other than those for culturing cells, and an air supply device for circulating gas.

For example, a fluid circuit device according to the aspect of the present invention may include the block that has the guide portion in which the pipe allowing a fluid sent from a pump to circulate therein is attachable and detachable. The guide portion may include at least one of the recessed portion that guides the pipe along the surface of the block, and the hole portion that guides the pipe through the inside of the block.

According to this constitution, since the pipe through which a fluid circulates and the block are segregated, even when the pipe is contaminated by a fluid, contamination of the block can be avoided and the block can be reused in terms of cost. Furthermore, when the pipe is contaminated, the contamination can be curbed by removing the pipe from the guide portion of the block and attaching an uncontaminated pipe to the guide portion. Therefore, costs can be reduced and contamination can be curbed.

Furthermore, when the pipe is attached to the guide portion of the block, since the pipe can be guided by at least one of the recessed portion of the block and the inside of the block, a situation in which the pipe is mixed up in a mess can be curbed. Therefore, occurrence of erroneous connection of the pipe or leakage of a fluid can be curbed and unnecessary use of a fluid due to an excessively long pipe can be curbed.

Each of the constituent elements described above as the embodiment or the modification examples thereof can be suitably combined within a range not departing from the gist of the present invention. In addition, it is also possible suitably not to use some constituent elements of a plurality of constituent elements combined together.

### BRIEF DESCRIPTION OF THE REFERENCE SYMBOLS

1 Cell culture device (fluid circuit device)
2 Block
3 Pump
4 Tank
5 Pipe
5A1 to 5A5 Pump connection tube (tube)
5B1 to 5B3 Relay tube (tube)
5C1 to 5C2 Coupling tube (tube)
6 Joint member
6A1 to 6A5 Pump joint tube (tube)
6B1 to 6B4 Relay joint tube (tube)
6C1 to 6C4 Coupling joint tube (tube)
9A to 9F, 109A to 109F First pipe connection system (pipe connection system)
9G to 9L, 209A to 209F Second pipe connection system (pipe connection system)
10 Guide portion
10A1 to 10A8 Pump-side guide portion
10B1 to 10B6 Relay guide portion
10C1 to 10C6 Coupling guide portion
10D1 to 10D2 Supply guide portion
11 Recessed portion
12 Hole portion
20, 120 Pump block (sub-block)
21 to 25 Pump cassette (cassette)
21a Pump mounting portion
30, 130, 330 Tank block (sub-block)
31 to 33 Tank cassette (cassette)
34 Supply cassette (cassette)
34a, 334a Tank mounting portion
35 Culture chip
40, 140, 240 Coupling block
41 to 43 Couple cassette (cassette)

## Claims

1. A fluid circuit device comprising:
a block that has a guide portion in which a pipe allowing a fluid sent from a pump to circulate therein is attachable and detachable,
wherein the guide portion includes at least one of
a recessed portion that guides the pipe along a surface of the block, and
a hole portion that guides the pipe through the inside of the block.

2. The fluid circuit device according to claim 1,
wherein the block is formed of a resin.

3. The fluid circuit device according to claim 1 or 2,
wherein the pipe is formed of a material with lower drug adsorption compared to the block.

4. The fluid circuit device according to any one of claims 1 to 3,
wherein the block includes a plurality of sub-blocks that are segregated in a manner of being attachable to and detachable from each other.

5. The fluid circuit device according to claim 4,
wherein the pipe includes a plurality of tubes that are provided correspondingly to the plurality of sub-blocks.

6. The fluid circuit device according to claim 5,
wherein the plurality of tubes include
a pump connection tube that is connectable to the pump, and
a tank connection tube that is connectable to a tank storing the fluid, and
the plurality of sub-blocks include
a pump block that has a pump-side guide portion in which the pump connection tube is attachable and detachable, and
a tank block that has a tank-side guide portion which is connected in a manner of being attachable and detachable with respect to the pump block and in which the tank connection tube is attachable and detachable.

7. The fluid circuit device according to claim 6,
wherein the pump block has a pump mounting portion in which the pump is mountable, and
the tank block has a tank mounting portion in which the tank is mountable.

8. The fluid circuit device according to claim 6 or 7,
wherein the plurality of tubes further include a coupling tube that allows the pump connection tube and the tank connection tube to be coupled,
the plurality of sub-blocks further include a coupling block that has a coupling guide portion in which the coupling tube is attachable and detachable, and
the coupling block is connected in a manner of being attachable and detachable with respect to the pump block and the tank block.

9. The fluid circuit device according to any one of claims 4 to 8,
wherein at least one of the plurality of sub-blocks includes a plurality of cassettes that are connected in a manner of being attachable to and detachable from each other, and
the pipe includes a plurality of tubes that are provided correspondingly to at least one of the plurality of cassettes.

10. The fluid circuit device according to any one of claims 5 to 9 further comprising:
a joint member that is provided at a part where the plurality of sub-blocks are connected to each other and is connected in a manner of being attachable and detachable with respect to the tubes.

11. A cell culture device comprising:
a block that has a guide portion in which a pipe allowing a cell culture fluid to circulate therein is attachable and detachable,
wherein the guide portion includes at least one of
a recessed portion that guides the pipe along a surface of the block, and
a hole portion that guides the pipe through the inside of the block.

12. The cell culture device according to claim 11,
wherein the block includes a plurality of coupling blocks that are coupled in a manner of being attachable to and detachable from each other, and
the plurality of coupling blocks have pipe connection systems that are different from each other in accordance with the purpose of cell culture.

13. The cell culture device according to claim 11 or 12,
wherein the block includes a supply cassette that has a supply guide portion in which a supply tube capable of supplying the cell culture fluid is attachable and detachable with respect to a culture chip capable of storing the fluid, and
the supply cassette is connected in a manner of being attachable and detachable with respect to an upper portion of the culture chip.
